# EUROPÄISCHE PATENTANMELDUNG

(11) **EP 2 439 276 A1**
(43) Veröffentlichungstag der Anmeldung: **11.04.2012**
(21) Anmeldenummer: 10186568.1
(22) Anmeldetag: 05.10.2010
(51) Int. Cl.: C12N 15/64, C12N 15/79

(54) **Verfahren zur semi-synthetischen Herstellung hochreiner "MiniCircle" DNA-Vektoren aus Plasmiden**

(71) Anmelder: Rentschler Biotechnologie GmbH, 88471 Laupheim (DE)
(72) Erfinder: Rehberger, Bernd, 88471 Laupheim (DE); Heine, Markus, 38124 Braunschweig (DE); Wodarczyk, Claas, 89231 Neu-Ulm (DE); Wagner, Roland, 88471 Laupheim (DE)
(74) Vertreter: Weiss, Wolfgang

(57) **Zusammenfassung**

Die vorliegende Erfindung betrifft Verfahren und Reagenzien zur Herstellung von DNA-Vektoren, insbesondere MiniCircle (MC) DNA-Vektoren, in superhelikaler Form. Weiterhin betrifft die Erfindung hochreine Präparationen von zirkulären DNA-Vektoren, insbesondere MC DNA-Vektoren.

SEQUENCE LISTING

<110> Rentschler Biotechnologie GmbH

<120> Verfahren zur semi-synthetischen Herstellung hochreiner
"MiniCircle" DNA-Vektoren aus Plasmiden.

<130> 48856P EP

<140> EP10186568.1
<141> 2010-10-05

<160> 6

<170> PatentIn version 3.5

<210> 1
<211> 20
<212> DNA
<213> Artificial Sequence

<220>
<223> Primer 3

<400> 1
ttttctgcgc gtaatctgct 20

<210> 2
<211> 19
<212> DNA
<213> Artificial Sequence

<220>
<223> Primer 4

<400> 2
gtaaaaaggc cgcgttgct 19

<210> 3
<211> 20
<212> DNA
<213> Artificial Sequence

<220>
<223> Primer 1

<400> 3
gcatgccatc atgacttcag 20

<210> 4
<211> 20
<212> DNA
<213> Artificial Sequence

<220>
<223> Primer 2

<400> 4
cgaaacgatc ctcatcctgt 20

<210>  5
<211>  480
<212>  DNA
<213>  Artificial Sequence

<220>
<223>  pEpi_delCM18opt_MC

<400>  5
tccccagcag gcagaagtat gcaaagcatg catctcaatt agtcagcaac caggtgtgga      60

aagtccccag gctccccagc aggcagaagt atgcaaagca tgcatctcaa ttagtcagca     120

accatagtcc cgcccctaac tccgcccatc ccgcccctaa ctccgcccag ttccgcccat     180

tctccgcccc atggctgact aatttttttt atttatgcag aggccgaggc cgcctcggcc     240

tctgagctat tccagaagta gtgaggaggc ttttttggag ggacggtatc gagcttgaag     300

ttcctatact ttctagagaa taggaacttc ggaataggaa cttcaccggt cgccaccatg     360

gtgagcaagg gcgaggagct gttcaccggg gtggtgccca tcctggtcga gctggacggc     420

gacgtaaacg gccacaagtt cagcgtgtcc ggcgagggcg agggcgatgc cacctacggc     480

<210>  6
<211>  460
<212>  DNA
<213>  Artificial Sequence

<220>
<223>  Sequenzierung

<400>  6
tccccagcag gcagaagtat gcaaagcatg catctcaatt agtcagcaac caggtgtgga      60

aagtccccag gctccccagc aggcagaagt atgcaaagca tgcatctcaa ttagtcagca     120

accatagtcc cgcccctaac tccgcccatc ccgcccctaa ctccgcccag ttccgcccat     180

tctccgcccc atggctgact aatttttttt atttatgcag aggccgaggc cgcctcggcc     240

tctgagctat tccagaagta gtgaggaggc ttttttggag ggacggtatc gagcttgaag     300

ttcctatact ttctagagaa taggaacttc ggaataggaa cttcaccggt cgccaccatg     360

gtgagcaagg gcgaggagct gttcaccggg gtggtgccca tcctggtcga gctggacggc     420

gacgtaaacg gccacaagtt cagcgtgtcc ggcgagggcg                           460

## Beschreibung

Die vorliegende Erfindung betrifft Verfahren und Reagenzien zur Herstellung von DNA-Vektoren, insbesondere MiniCircle (MC) DNA-Vektoren, in superhelikaler Form. Weiterhin betrifft die Erfindung hochreine Präparationen von zirkulären DNA-Vektoren, insbesondere MC DNA-Vektoren.

Bisher verwendete Verfahren zur Herstellung der zirkulären MiniCircle DNA-Vektoren sind gekennzeichnet durch hohe Komplexität bei niedrigen Ausbeuten und großem Aufwand zur Erreichung der notwendigen Reinheit und Qualität (1, 3, 8, 11, 15).

Diesen Verfahren ist gemein, dass sie Rekombinasen einsetzen, um die MC aus den zuvor in Bakterien vermehrten Parentalplasmiden auszuschneiden. Einerseits garantiert der Einsatz der Rekombinasen den Erhalt des superhelikalen Status des MC, der für den weiteren Einsatz als Vektor zur Transduktion eukaryontischer Zellen notwendig ist. Andererseits ist dieser Vorgang extrem ineffizient, so dass die Ausbeute an MC, bezogen auf die Menge an eingesetzter Bakterienkultur, sehr gering ist. Dies liegt zu einem entscheidenden Teil daran, dass eine permanente Expression der für die Generierung der MC nötigen Rekombinasen in den Produktions-Bakterienstämmen nicht möglich ist, da dies zu einer frühzeitigen Eliminierung der in den Bakterien replizierenden Parentalplasmide führen würde, bevor diese in ausreichender Menge amplifiziert werden konnten, um als Ausgangssubstrat für die Herstellung der MC zur Verfügung zu stehen.

Da der Erhalt der superhelikalen Struktur für den späteren Einsatz der MC essenziell ist, sind auch die Anforderungen an die Aufreinigungsmethode zur Isolierung der MC entsprechend hoch, um die gewünschte Struktur der MC während der Aufreinigung nicht zu zerstören. In der klassischen Variante ((1), (3)) erfolgt die Abtrennung der MC von den nach der Rekombination entstandenen DNA-Nebenprodukten - Parentalplasmid, Miniplasmid und Konkatemeren der einzelnen Produkte - durch eine gezielte Linearisierung der ungewünschten Moleküle mittels speziell ausgewählter Restriktionsenzyme und anschließender Agarose-Gelelektrophorese. Der MC wird dabei von den Restriktionsenzymen nicht modifiziert, so dass der zirkuläre Status der MC nicht beeinflusst wird. Dabei besteht jedoch ein signifikantes Risiko, unerwünschte DNA Moleküle wie z.B. das (evtl. linearisierte) Parentalplasmid zusammen mit den MC aus dem Gel zu eluieren und aufzureinigen (3). Da auch die Parentalplasmide wie die MC in der Lage sind, eukaryontische Zellen zu transduzieren, kann dies für das MC-Experiment negative Folgen haben (z.B. dauerhaft transduzierte Zellen auf Grund der Integration des Parentalplasmids ins Genom der Wirtszelle).

Andere Varianten zur Aufreinigung von durch Rekombination in Bakterien generierten MC verwenden ein säulenchromatographisches System, das sich die Bindung spezieller Proteine an die in diesem Fall im MC enthaltene Sequenz *des Lactose-Operators (lacOs)* zu Nutze macht (11, 12). In diesem Fall sind die erzielte Reinheit und auch die relative Ausbeute des Systems besser als in der klassischen Variante der Aufreinigung über das Agarosegel. Dafür muss der Nachteil in Kauf genommen werden, dass der MC dann bakterielle DNA Sequenzen *(IacOs)* enthält, was den funktionellen (5, 6) und regulatorischen Vorteil der MC gegenüber den klassischen Plasmid-Vektoren untergräbt.

Durch das in der vorliegenden Erfindung vorgestellte Verfahren zur Herstellung von MC *in vitro* können die Nachteile der bisher veröffentlichten Methoden zur MC Herstellung überwunden werden.

Das neue Verfahren verzichtet auf den Einsatz von Rekombinasen zur Generierung der MC aus dem Parentalplasmid. Gleichzeitig ist es nicht nötig, die erhaltenen MC über komplexe und teure Verfahren wie z.B. der Säulenchromatografie aufzureinigen, da eine Vermischung der MC mit den verschiedenen Nebenprodukten von vornherein vermieden wird. Dies führt zu einer Vereinfachung der Herstellung von MC gegenüber den bisher gängigen Verfahren bei gleichzeitiger Erhöhung von Ausbeute und Reinheit der Zielmoleküle, zumal überflüssige bakterielle Sequenzen im Rückgrat des MC komplett vermieden werden.

Das hier vorgestellte Verfahren basiert auf einer kombinierten *in vivo*/*in vitro-*Technologie, bei der in einem finalen enzymatischen Schritt mit Hilfe von Gyrase zirkuläre superhelikale MiniGircle-DNA generiert wird.

Ein Gegenstand der vorliegenden Erfindung ist somit ein Verfahren zur Herstellung eines zirkulären DNA-Vektors in superhelikaler Form, umfassend die Schritte:
(a) Spalten eines Parentalplasmids mit einem oder mehreren Restriktionsenzymen, wobei das Parentalplasmid die Sequenz des DNA-Vektors sowie heterologe Sequenzen enthält, um ein lineares DNA-Vektor-Fragment mit der Sequenz des DNA-Vektors zu erhalten,
(b) Abtrennen des linearen DNA-Vektor-Fragments von anderen Produkten der Reaktionsspaltung,
(c) Ligieren des linearen DNA-Vektor-Fragments, um einen zirkulären DNA-Vektor in relaxierter Form zu erhalten,
(d) Abtrennen des zirkulären DNA-Vektors von anderen Produkten der Ligation,
(e) Verdrillen des zirkulären DNA-Vektors aus Schritt (d) mit einer Gyrase, um einen zirkulären DNA-Vektor in superhelikaler Form zu erhalten, und
(f) gegebenenfalls Aufreinigen des zirkulären DNA-Vektors in superhelikaler Form zur Abtrennung von Nebenprodukten.

Noch ein weiterer Gegenstand der Erfindung ist ein Verfahren zur Herstellung von superhelikalen zirkulären DNA-Vektoren, umfassend eine Restriktionsspaltung *in vitro,* eine Ligation *in vitro* und eine Verdrillung mit Gyrase *in vitro.*

Noch ein weiterer Gegenstand der Erfindung ist ein Verfahren zur Herstellung von superhelikalen MiniGircle DNA-Vektoren ohne den Einsatz orts-(sequenz-) spezifischer Rekombinasen wie etwa FLP.

In einer bevorzugten Ausführungsform erfolgt die Herstellung des DNA-Vektors, insbesondere des MC-DNA-Vektors, wie folgt:
- Die benötigten Vektorinformationen werden zusammen mit heterologen Sequenzen auf dem Parentalplasmid (PP) in einer Wirtszelle, z.B. in einem Bakterium, amplifiziert.
- Das Parentalplasmid wird mit Standardmethoden aus der Wirtszelle isoliert.
- Nach der Isolierung werden die MC-Sequenzen des PP mittels Verdau mit Restriktionsenzymen aus dem PP ausgeschnitten. Die dadurch entstandenen linearen Fragmente, von denen eines die Bereiche des MC enthält, werden z.B. durch Agarosegelelektrophorese aufgetrennt. Die linearisierten MC Fragmente werden mit gängigen Methoden isoliert, z.B. durch Elution, und für die weitere MC Herstellung verwendet.
- Die isolierten linearisierten MC-DNA-Moleküle werden durch die Verwendung (rekombinanter) Ligasen religiert, so dass die MC in der relaxierten, sog. "open circle" Form entstehen.
- Im Anschluss an die Ligation werden die relaxierten MC unter Einsatz (rekombinanter) Gyrase in einem *in vitro*-Ansatz verdrillt, so dass die entsprechenden superhelikalen (supercoiled) Formen der MC entstehen, die für deren Einsatz zur Transduktion eukaryontischer Zellen benötigt werden.
- Abschließend wird der zirkuläre, superhelikale Vektor mit Standardmethoden (z.B. Agarose Gelelektrophorese mit anschließender Gel-Elution, Qiagen DNA Aufreinigungskits) von den Beiprodukten der enzymatischen Gyrase-Reaktion gereinigt.

Durch das hier beschriebene Verfahren zur Herstellung von DNA-Vektoren, insbesondere von MiniCircle-Genvektoren wird es ermöglicht, die z.B. für die Herstellung rekombinanter Zelllinien oder die Gentherapie einsetzbaren DNA-Vektoren mit einfachen Methoden in großer Menge und Reinheit herzustellen.

Außerdem werden durch das erfindungsgemäße Verfahren neuartige Präparationen von MC-Vektoren in hoher Reinheit erhalten.

Dies führt zum Einen zu einer deutlichen Reduktion an Kosten und Zeit für die bisher beschriebenen Einsatzgebiete und eröffnet zudem auch Perspektiven für neue Anwendungsfelder.

Gemäß dem o.g. Verfahren wird ein zirkulärer DNA-Vektor in superhelikaler Form hergestellt. Der DNA-Vektor besteht aus doppelsträngiger DNA und hat üblicherweise eine Größe von 0,5 bis etwa 10 kB, insbesondere von ca. 1-6 kB. Es können jedoch auch kleinere oder größere DNA-Vektoren hergestellt werden. In einer bevorzugten Ausführungsform ist der DNA-Vektor ein MiniCircle (MC) DNA-Vektor, d.h. ein zirkulärer DNA-Vektor, der die für den DNA-Vektor, z.B. den späteren MiniGircle DNA-Vektor, nötigen Funktionen enthält. Diese Funktionen beinhalten üblicherweise Sequenzen für ein Transgen, z.B. ein rekombinantes Gen oder eine entsprechende cDNA, zusammen mit regulatorischen Sequenzen zur Genexpression, z.B. Transkriptions- und Translations-Initiationssequenzen wie Promotoren, Enhancer, ribosomale Bindungsstellen, etc. und ggf. Transkriptions-Terminations-Sequenzen wie polyA Sequenzen und weitere regulatorische oder funktionelle Nucleotid-Sequenzen wie z.B. S/MAR Sequenzen zur Übertragung der Adhärenz des DNA-Vektors an die Kernmatrix als Auslöser für integrationsunabhängige episomale Replikation (2, 13, 14).

Als Transgen kann der DNA-Vektor eine pharmazeutisch verwendbare DNA-Sequenz enthalten, z.B. ein Gen bzw. eine cDNA aus Säugern, insbesondere ein humanes Gen oder eine rekombinante Variante davon für therapeutische Anwendungen, oder eine synthetische Sequenz, z.B. ein synthetisches Gen. Eine solche Sequenz kodiert z.B. für Wachstumsfaktoren, Cytokine, Interleukine, Interferone, Tumorsuppressor-Proteine etc. Andererseits kann der DNA-Vektor als Transgen auch eine Sequenz aus pathogenen Organismen, die für ein Antigen kodiert, oder eine Sequenz, die für ein Tumor- oder Autoantigen kodiert, enthalten.

Die durch das erfindungsgemäße Verfahren erhältlichen Präparationen von zirkulär-superhelikalen DNA-Vektoren, insbesondere MC Vektoren, können sowohl als Forschungsreagenzien als auch als pharmazeutische Produkte verwendet werden. Bevorzugt sind medizinische Anwendungen wie etwa DNA Vakzinierung,Gentherapie, Neu- oder Reprogrammierung von Zellen oder der Einsatz von RNAi.

Darüber hinaus können die aus dem Verfahren hergestellten DNA-Vektoren, insbesondere MC Vektoren, auch zur Herstellung von therapeutischen Proteinen in rekombinanten Zellen, insbesondere eukaryontischen Zellen, insbesondere CHO Zellen, verwendet werden. Dazu werden die entsprechenden Wirtszellen transient oder stabil durch die DNA-Vektoren transduziert, so dass die daraus resultierenden rekombinanten Zellen das gewünschte therapeutische Protein produzieren. Die Produktion des therapeutischen Proteins kann dabei unter gängigen industriellen biotechnologischen Methoden erfolgen.

Ein weiteres Einsatzgebiet der mit dem hier beschriebenen Verfahren hergestellten DNA-Vektoren, insbesondere MiniCircle Vektoren, ist die genetische Modifikation von Wirtszellen, die zur Expression rekombinanter Proteine verwendet werden sollen. Dabei werden die Wirtszellen durch die DNA-Vektoren so modifiziert, dass die Expression des transgenen Proteins in den modifizierten Wirtszellen in Quantität und/oder Qualität der Proteinexpression aus unveränderten Wirtszellen überlegen ist.

Zudem ermöglicht der Einsatz der DNA-Vektoren, insbesondere MC-Vektoren, die nachträgliche Modifikation von bereits rekombinantes Protein produzierenden Produktionszellen, so dass diese durch die durch den MC eingebrachten Gene in gewünschter Weise bzgl. Transgen-Quantität und/oder Qualität moduliert werden können.

Um die entsprechenden Zielzellen mit den durch das beschriebene Verfahren hergestellten MC zu verändern, kann die Transduktion der Zielzellen mit einem oder mehreren MC gleichzeitig erfolgen.

Die Generierung der zirkulären superhelikalen DNA-Vektoren erfolgt aus sogenannten Parentalplasmiden, die sich in ihren Grundfunktionen nicht von gewöhnlichen Plasmiden unterscheiden. Sie dienen zur Amplifikation der DNA-Vektorsequenzen in Wirtszellen, z.B. Bakterien wie etwa E.coli. Andere Beispiele für geeignete Wirtszellen sind Hefen, wie etwa Saccharomyces. Diese Parentalplasmide tragen neben der Sequenz des DNA-Vektors üblicherweise auf einem zusammenhängenden Teil des Gesamtplasmids heterologe Sequenzen, z.B. zur Propagation in einer Wirtszelle, wie etwa Informationen für den Replikationsursprung zur Initiierung der Replikation des Plasmids in Wirtszellen sowie im Normalfall eines Gens inklusive regulatorischer Sequenzen zur Übertragung einer Antibiotikum-Resistenz auf die Plasmid tragenden Wirtszellen.

Da die Herstellung von DNA-Vektoren gemäß vorliegender Erfindung ohne durch orts- bzw. sequenzspezifische Rekombinasen vermittelte DNA-Rekombination auskommt, kann ein Parentalplasmid als Ausgangsmaterial verwendet werden, das frei von Rekombinase-Erkennungssequenzen, z.B. frei von Erkennungssequenzen für sequenzspezifische Rekombinasen, wie etwa FLP, Cre, RecA, Phi-C31 und weiteren ist.

Vorzugsweise besteht das Parentalplasmid (PP) daher im Wesentlichen aus zwei Teilen:
- Dem Teil mit den funktionellen Einheiten zur Replikation und Amplifikation des Parentalplasmids in Wirtszellen, z.B. Bakterien.
- Dem zweiten Teil mit den Informationen, die für den DNA-Vektor benötigt werden.

Getrennt werden die Plasmid- und die DNA-Vektorsequenzen durch Erkennungssequenzen für ein oder mehrere Restriktionsenzyme, die vorzugsweise nicht auf dem DNA-Vektor-Fragment vorkommen.

Das als Ausgangsmaterial des erfindungsgemäßen Verfahrens verwendete Parentalplasmid wird üblicherweise durch Kultivierung einer Wirtszelle, insbesondere einer prokaryontischen Wirtszelle, wie etwa E.coli, und Isolierung des Plasmids aus de Wirtszelle, gewonnen. Vorzugsweise wird als Wirtszelle ein Bakterienstamm verwendet, welcher zur High-Copy-Amplifikation von Plasmiden geeignet ist, wie etwa E.coli XL1 Blue (16). Die Gewinnung von Parentalplasmiden unterscheidet sich dabei nicht von Verfahren zur Herstellung üblicher Plasmide oder DNA-Vektoren.

Die Aufreinigung der PP aus den Bakterien kann nach Standardmethoden, z.B. mit kommerziell erhältlichen Kits (z.B. QlAgen Midiprep), erfolgen.

Schritt (a) des erfindungsgemäßen Verfahrens umfasst die Spaltung eines Parentalplasmids mit einem oder mehreren Restriktionsenzymen. Dieser Schritt wird günstigerweise *in vitro, d.h.* an einer isolierten Plasmid-Präparation durchgeführt. Zur Spaltung des Parentalplasmids werden Restriktionsenzyme verwendet, die ein Herausschneiden eines Fragments ermöglichen, welches die Sequenz des DNA-Vektors umfasst (DNA-Vektor-Fragment). Dieses DNA-Vektor-Fragment wird in linearer Form neben einem oder mehreren weiteren linearen Fragmenten entsprechend den im Parentalplasmid vorhandenen, zusätzlichen, heterologen Sequenzen erhalten.

Bei der Restriktionsspaltung wird vorzugsweise der Bereich des DNA-Vektors nicht geschnitten, während die verbleibende Sequenz, auch MiniPlasmid (MP) bezeichnet, entweder als ganzes Stück zurückbleibt, oder aber durch die Spaltung in kleinere Stücke zerlegt wird. Wichtig dabei ist, dass keine DNA-Fragmente entstehen, die in der Größe ähnlich zum DNA-Vektor-Fragment sind. Die Restriktionsspaltung läuft vorzugweise quantitativ ab, so dass nach der enzymatischen Behandlung der DNA kein inaktes Parentalplasmid mehr vorliegt.

Gemäß Schritt (b) wird das lineare DNA-Vektor-Fragment von anderen Produkten der Restriktionsspaltung, d.h. anderen linearen DNA-Fragmenten, abgetrennt. Üblicherweise erfolgt die Abtrennung nach Größe, z.B. durch Gelelektrophorese. Bevorzugt ist eine Abtrennung durch Agarose-Gelelektrophorese. Als Ergebnis der Abtrennung wird das lineare DNA-Vektor-Fragment in hochreiner Form, frei von anderen Produkten der Restriktionsspaltung, isoliert. Da das DNA-Vektor-Fragment in linearisierter Form vorliegt, kann es anhand seiner Größe mittels eines gewöhnlichen DNA-Markers im Gel leicht identifiziert werden.

Der linearisierte MC kann nach Standardmethoden isoliert werden, z.B. durch Elution und Reinigung mittels kommerzieller Kits (z.B. QlAgen Gel elute) aus dem Agarosegel. Die so erhaltene DNA enthält nur linearisierte MC-DNA.

Gemäß Schritt (c) wird das lineare DNA-Vektor-Fragment mit einer Ligase in Kontakt gebracht, unter Bedingungen, unter denen eine Ligation erfolgt, wobei ein zirkulärer DNA-Vektor in relaxierter Form erzeugt wird. Schritt (c) wird günstigerweise *in vitro,* d.h. an einer isolierten Präparation des DNA-Vektor-Fragments durchgeführt. Geeignete Ligasen sind kommerziell, z.B. in rekombinanter Form, erhältlich.

Um die Ligation möglichst effektiv zu gestalten, sollte bei dem zuvor durchgeführten Verdau des PP darauf geachtet werden, dass die dabei entstehenden 5'- und 3'-Enden des linearisierten DNA-Vektor-Fragments möglichst über komplementäre Nuecleotid-Überhänge verfügen. Gegebenenfalls kann auch eine Ligation mit glatten Enden durchgeführt werden.

Um zu verhindern, dass unter Umständen mitgeschleppte linearisierte MP-DNA zirkularisiert wird bzw. sich aus linearisiertem MC und linearem MP wieder ein PP entsteht, ist es bevorzugt, bei der Auswahl der Restriktionsenzyme zur Trennung von MC und MP darauf zu achten, dass das MP mittels eines zusätzlichen Enzyms so geschnitten wird, dass kein vollständiges MP-Fragment entsteht, das mit sich selbst oder mit dem MC zirkularisiert werden kann.

Nach der Ligation wird der Ansatz gemäß Schritt (d) aufgereinigt, um den zirkulären DNA-Vektor von anderen Produkten der Ligation abzutrennen. Dies kann z.B. durch Auftrennung über ein Agarosegel. Hier werden evtl. Kontaminationen mit MP-Fragmenten oder MC-Konkatemere sichtbar. Aus dem Gel wird nur die Bande für den zirkularisierten DNA-Vektor (MC) ausgeschnitten und daraus die DNA eluiert. Dadurch wird der Reinheitsgrad der MC-DNA nochmals erhöht und eine Kontamination mit MP- oder PP-Fragmenten praktisch ausgeschlossen.

Für den Einsatz des MC als DNA-Vektor - speziell bei seiner Verwendung als stabil episomal replizierender Vektor - ist es notwendig, dass das ringförmige DNA-Molekül in superhelikaler Form vorliegt. Dieser superhelikale Status ist nach erfolgter Zirkularisierung mit Ligase nicht gegeben. Deshalb muss dieser nachträglich mittels Gyrase-Behandlung der zirkulären MC hergestellt werden. Bei der Gyrase - die in rekombinanter Form kommerziell erhältlich ist - handelt es sich um eine Typ II Topoisomerase, die in Anwesenheit von ATP die Einführung negativer superhelikaler Strukturen in DNA bewirkt.

Schritt (e) umfasst daher das Inkontaktbringen des zirkulären DNA-Vektors aus Schritt (d) mit einer Gyrase unter Bedingungen, bei denen eine Verdrillung des Vektors erfolgt, z.B. in Gegenwart von ATP. Auf diese Weise wird ein zirkulärer DNA-Vektor in superhelikaler Form in hoher Reinheit und Ausbeute erhalten. Schritt (e) wird günstigerweise *in vitro,* d.h. an einer isolierten Präparation des DNA-Vektors durchgeführt. Die Reaktionsdauer kann variiert werden, um Präparationen mit unterschiedlichem Verdrillungsgrad zu erhalten.

Die Reaktion kann nach den Vorschriften des Herstellers zur Anwendung der Gyrase erfolgen. Nach Einführung der superhelikalen Strukturen in die MC können diese abschließend mittels Standardmethoden, z.B. mittels kommerzieller Kits (z.B. QlAgen MidiPrep) aus dem Reaktionsansatz aufgereinigt werden.

Schritt (e) umfasst die Aufreinigung der zirkulären, superhelikalen DNA-Vektoren mittels Standardmethoden (Fällung, Agarosegelelektrophorese mit anschließender Elution, Qiagen Qiaquick Nucleotide Removal Kit etc.) zur Abtrennung von Nebenprodukten aus der enzymatischen Gyrase-Reaktion.

Nach der Herstellung der MC mit der hier beschriebenen Methode, gibt es mehrere Möglichkeiten, den Erfolg und die Qualität der MC-Präparation zu testen:
- Agarosegel-Elektrophorese. Hier darf nur eine Bande zu sehen sein, deren Größe mit Hilfe eines DNA-Markers für superhelikale DNA bestimmt werden kann.
- Überprüfung der superhelikalen Struktur, z.B. anhand eines Chloroquin-Gels. Mit einem Gel dieser Art kann zirkuläre DNA je nach Anzahl der eingeführten superhelikalen Strukturen in ihre verschiedenen Formen aufgetrennt werden (10).
- Mittels PCR kann mit sehr hoher Sensitivität überprüft werden, ob ausschließlich zirkuläre MC-Strukturen in den Präparationen vorliegen, oder ob es auch Kontaminationen mit linearem oder zirkulärem MP und/oder PP gibt.

Ein weiterer Gegenstand der Erfindung ist ein Reagenzienkit, welcher Restriktionsenzyme, eine Ligase und eine Gyrase zur Durchführung des erfindungsgemäßen Verfahrens enthält.

Noch ein weiterer Gegenstand der Erfindung ist eine Präparation eines DNA-Vektors, insbesondere eines MiniCircle DNA-Vektors in superhelikaler Form gekennzeichnet durch das Fehlen von Nebenprodukten, insbesondere linearem oder zirkulärem Miniplasmid und/oder Parentalplasmid. Vorzugsweise enthält die Präparation nach PCR keinen Hinweis auf die o.g. Nebenprodukte.

Um mittels PCR verunreinigende Parentalplasmide nachzuweisen, müssen die sequenzspezifischen Primer so gewählt werden, dass eines der beiden Oligonucleotide im Bereich des heterologen Rückgrats des Parentalplasmids bindet, während der korrespondierende Primer sich in der Region des MiniCircles befindet. Bei dieser Anordnung der Oligonucleotide kommt es nur zu einer Amplifikation des entsprechenden Fragments, wenn in der MC Präparation Parentalplasmid vorkommt. Mit Hilfe einer zweiten PCR können auch kontaminierende Miniplasmide gefunden werden. Dazu müssen beide PCR-Primer im Bereich des heterologen Rückgrats des ursprünglichen Parentalplasmids liegen. Kommt es zur Amplifikation des PCR-spezifischen Fragments, so kann diese entweder von Miniplasmiden oder dem Parentalplasmid stammen. War die erste PCR negativ für Parentalplasmid, so ist das Amplifikat der zweiten PCR auf Miniplasmide zurück zu führen. Sind beide PCR Reaktionen positiv, so ist keine eindeutige Aussage hinsichtlich der Art der Nebenprodukte möglich.

Die *in vitro* hergestellten MiniCircles verfügen über gezielt steuerbare superhelikale Strukturanteile. Die dadurch erreichte Vorhersagbarkeit der Zusammensetzung der *in vitro* MC Präparation ist der zufälligen Zusammensetzung von aus Bakterien durch ortsspezifische Rekombinantion gewonnenen zirkulären DNA-Molekülen deutlich überlegen. Diese Überlegenheit hat substanzielle Konsequenzen, insbesondere für den Einsatz therapeutischer Vektoren bei klinischen Anwendungen.

Weiterhin soll die vorliegende Erfindung durch die nachfolgenden Figuren und Beispiele erläutert werden.

### Figurenlegende

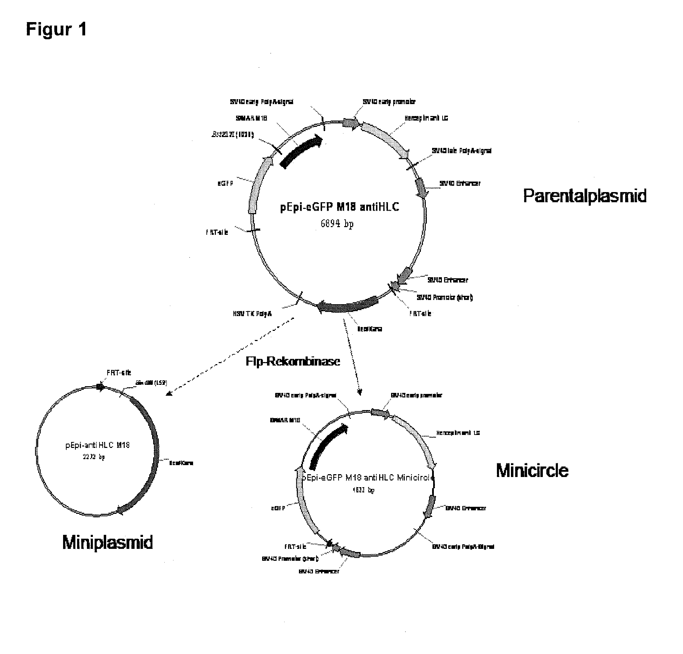

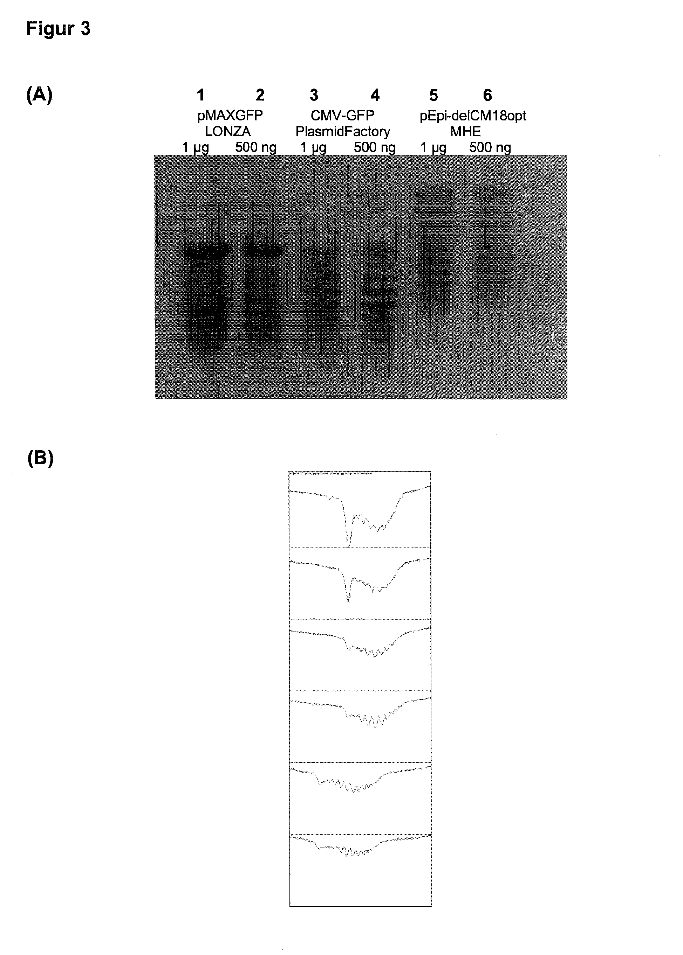

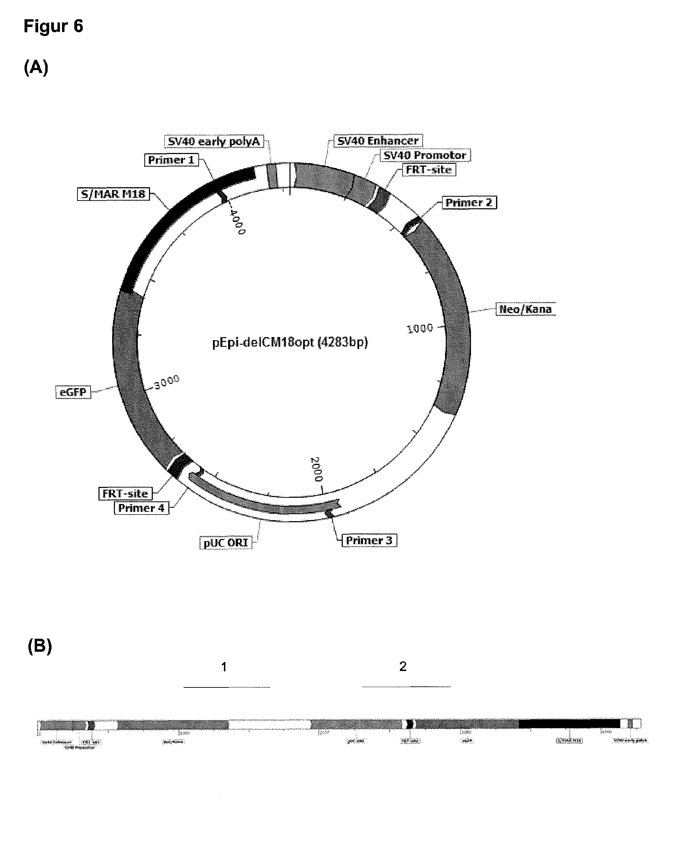

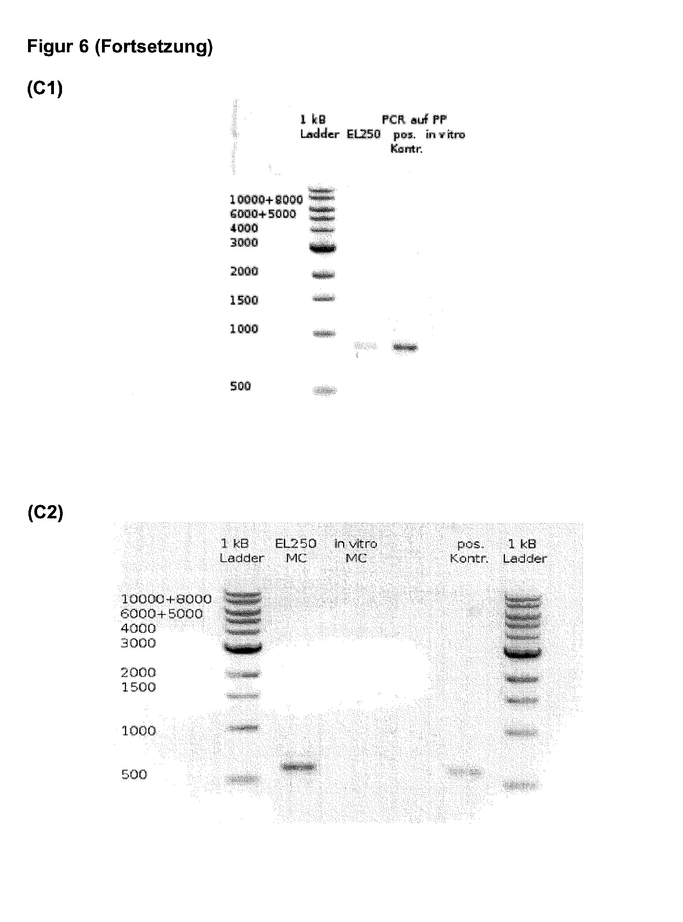

**Figur 1****: Plasmidkarte des MC Parentalplasmides "pEpi-eGFP M18 antiHLC" und der Rekombinationsprodukte.**
   Das Parentalplasmid enthält alle für ein funktionierendes Plasmid notwendigen Einheiten wie Origin of Replication (ori, in diesem Falle zwischen HSV TK polyA und der folgenden FRT-Site; nicht eingezeichnet) und Antibiotikum-Resistenz (Neo/Kana). Entscheidend zur Herstellung von MC-Vektoren nach der gängigen Methode mittels sequenzspezifischer Rekombination ist das Vorhandensein der beiden FRT-Sequenzen, die als Angriffspunkte für die FLP-Recombinase dienen. Durch die FLP-induzierte Rekombination dieser beiden Sequenzen gegeneinander kommt es hier zur Abschnürung zweier unabhängiger zirkulärer DNA-Moleküle. Eines davon trägt die Informationen für die in Bakterien wichtigen Funktionen und wird deshalb als MiniPlasmid bezeichnet. Das zweite Molekül enthält keine funktionalen bakteriellen Sequenzen mehr, sondern trägt nur noch die für den Vektor spezifischen Informationen und stellt somit den MiniCircle dar.

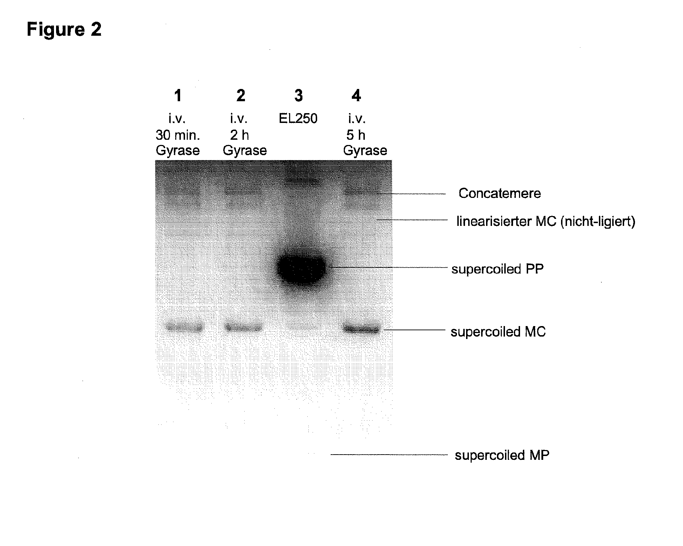

**Figur 2****: Vergleich des *"in vitro*"-Minicircle mit dem per ortsspezifischer Rekombination in *E. coli* EL250 hergestellten MiniCircle mithilfe eines 1 %-igen Agarosegels.**
   Der *in vitro*-MC wurde durch Restriktion mit dem Enzym Xbal und anschließender Re-Ligation mit einer T4-Ligase aus dem PP erhalten. Anschließend wurde die ligierte DNA (je 1 µg) für 30 min. (Spur 1), 2 h (Spur 2) oder 5 h (Spur 4) mit 1 U DNA-Gyrase behandelt. Der "EL250"-MC wurde durch ortsspezifische Rekombination in *E. coli* EL250 nach Induktion mit 0,3 % L-Arabinose erhalten (Spur 3). Die *in vitro*-MC und die "EL250"-MC besitzen in einem 1 % Agarosegel dasselbe Laufverhalten, d.h. dass mit Hilfe der DNA-Gyrase in die ligierte MC-DNA superhelikale Strukturen eingeführt wurden.
   In der Spur 1, 2 und 4 ist erkennbar, dass bereits nach 30 min GyraseBehandlung der ligierte MC vollständig in die supercoiled (ccc)-Form überführt wurde. Weiterhin sind MC-Concatemere und nicht-ligierte MC-DNA zu erkennen. In der Spur 3 sind zusätzlich zur MC-Bande eine starke PP-Bande und eine MP-Bande zu erkennen.
   Legende: i.v. = *in vitro*-MiniCircle; EL250 = MiniCircle, durch ortsspezifische Rekombination in *E. coli* EL250 hergestellt; MC= MiniCircle; PP = Parentalplasmid; MP = Miniplasmid
**Figur 3****: Auftrennung verschiedener ccc-Plasmide auf einem 0,8 % Chloroquin-Agarosegel**
   Es wurden die Plasmide pMAXGFP (LONZA), CMV-GFP-Parentalplasmid und pEpi-delCM18opt (Rentschler Biotechnologie) verwendet. Das Agarosegel enthielt 2,5 µg mL⁻¹ Chloroquin. Der Gellauf erfolgte für 15 h bei 2,5 V cm⁻¹. (A) Je Plasmid wurden entweder 1 µg oder 500 ng für den Gellauf verwendet. Bei allen verwendeten Plasmiden ist ein charakteristisches Bandenmuster entstanden. (B) Die Signale wurden mit dem Programm ImageJ quantifiziert.

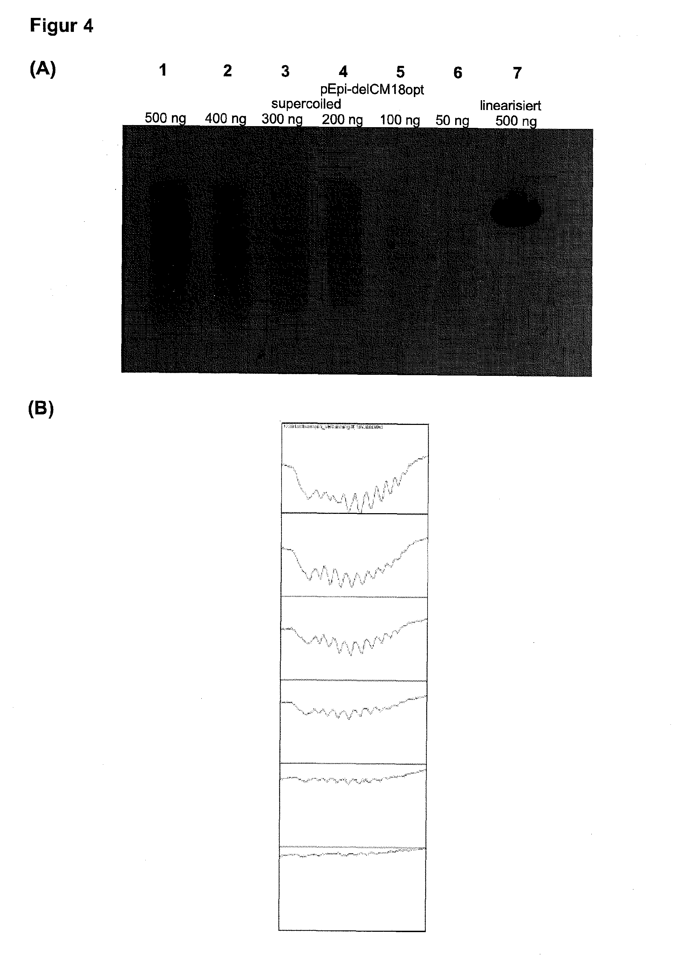

**Figur 4****: Auftrennung verschiedener Mengen des ccc-Plasmid pEpidelCM18opt auf einem 0,8 % Chloroquin-Agarosegel.**
   Die DNA wurde für 15 h bei 2,5 V cm⁻¹ aufgetrennt. Das Gel enthielt 2,5 µg mL⁻¹ Chloroquin. (A) Die Reihe 1 - 6 enthält verschiedene Mengen an ccc-DNA, die Reihe 7 enthält das linearisierte Plasmid. (B) Die Bandenmuster wurden mit dem Programm ImageJ aufgenommen. Es wird deutlich, dass der Auftrag einer Menge von 100 ng DNA für die Auswertung der Bandenmuster ausreichend ist. Bei längerer Belichtung können sogar noch 50 ng ausgewertet werden (Daten nicht gezeigt).

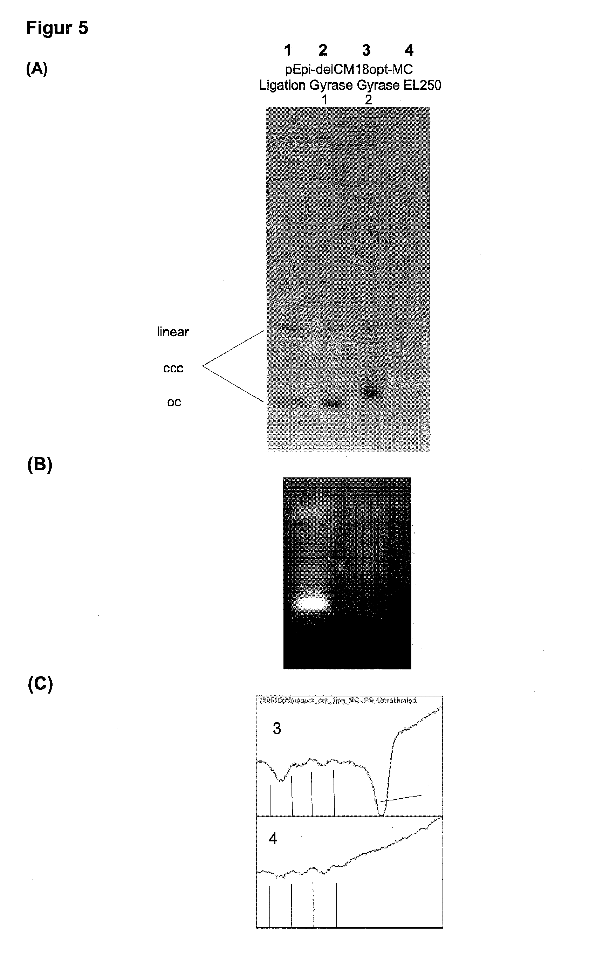

**Figur 5****: Vergleich der Verdrillung des "*in vitro*"-MiniCircles mit dem per ortsspezifischer Rekombination entstandenen MiniCircle aus dem Plasmid pEpi-delCM18opt**
   (A) Spur 1: Der *in vitro* MiniCircle wurde durch Restriktionsverdau des Parentalplasmids mit den Enzymen Xbal und BstBI und anschließender Ligation (T4-Ligase) hergestellt (oc). Es ist zu erkennen, dass die Ligation nicht vollständig ist, ein gewisser Teil der DNA bleibt linearisiert. Weiterhin entstehen Concatemere aus zwei oder mehreren DNA-Stücken. Spur 2: Die Gyrase und der Gyrasepuffer wurden direkt dem Ligationsansatz (1 µg DNA) hinzugefügt. Es ist erkennbar, dass keine Verdrillung stattgefunden hat. Spur 3: Die Ligation wurde zunächst aufgereinigt (QIAGEN PCR Purification Kit), anschließend wurde 1 µg ligierte DNA für 2 h mit 5 U Gyrase behandelt. Spur 4: Der MiniCircle wurde per ortsspezifischer Rekombination hergestellt. Diese wurde in *E. coli* EL250 mit Hilfe einer im Genom kodierten FLP-Rekombinase durch L-Arabinose induziert.
   (B) Die Spur 3 und 4 aus (A) wurden hier vergrößert und invertiert dargestellt. Der per ortspezifischer Rekombination entstandene MC (Spur 4) zeigt das erwartete Bandenmuster. Der *in vitro* MC (Spur 3) zeigt ebenfalls das Bandenmuster, eine Bande ist jedoch besonders ausgeprägt.
   (C) Die Signale wurden mit dem Programm ImageJ aufgenommen und sind entsprechend mit Pfeilen gekennzeichnet. Bei der Spur 3 ist das in (B) erwähnte deutliche Bandensignal zu erkennen (roter Pfeil).
   Legende: linear= linearisierte DNA, oc = open circle DNA, ccc= supercoiled DNA, U= Unit, EL250 = per ortsspezifischer Rekombination hergestellter MC, MC= MiniCircle
**Figur 6****: Überprüfung der Reinheit der generierten MiniCircle per PCR und 1 % Agarosegel**
   Um die generierte MiniCircle-DNA aus pEpi-delCM18opt (Vektorkarte A) auf mögliche Verunreinigungen mit Parentalplasmid oder Miniplasmid zu überprüfen, wurde per PCR ein Stück in der Miniplasmid-Region (s. linearisierte Vektorkarte B; PCR 1) und über eine FRT-Site (s. linearisierte Vektorkarte B, PCR 2) amplifiziert. Es wurde jeweils 10 ng Template-DNA verwendet. Entsteht bei der PCR 1 ein Amplifikat, enthält die Probe entweder Miniplasmid oder Parentalplasmid, entsteht bei der PCR 2 ein Amplifikat, enthält die Probe Parentalplasmid, da bei dieser PCR ein Primer in der MiniCircle-Region und ein Primer in der Miniplasmid-Region liegt. (C1) In der Probe mit dem per ortsspezifischer Rekombination hergestellten MiniCircle (EL250 MC) ist ein Amplifikat entstanden, in der Probe mit dem In-vitro-MiniCircle ist kein Amplifikat entstanden. (C2) Es ist ebenfalls bei dem EL250 MiniCircle ein Amplifikat entstanden. Legende: PP = Parentalplasmid; MC = Minicircle, EL250 MC= MiniCircle, der per ortsspezifischer Rekombination im *E. coli*-Stamm EL250 hergestellt wurde.

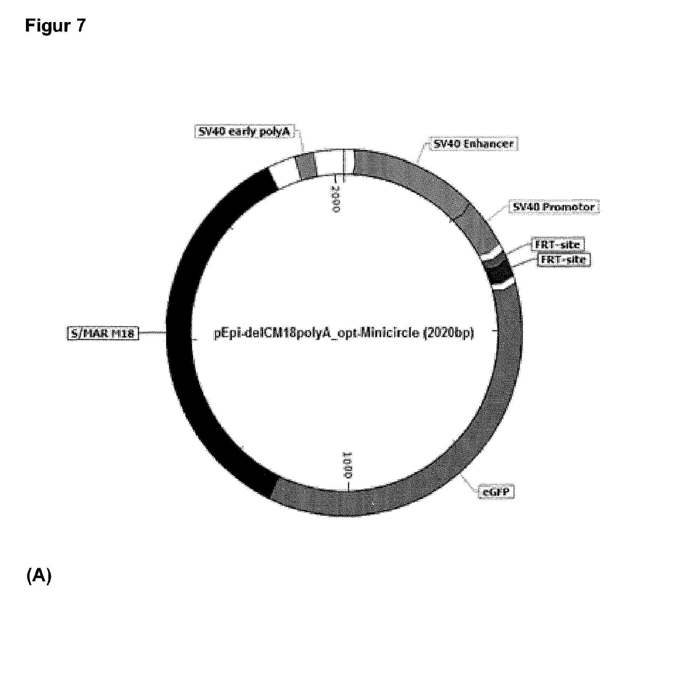

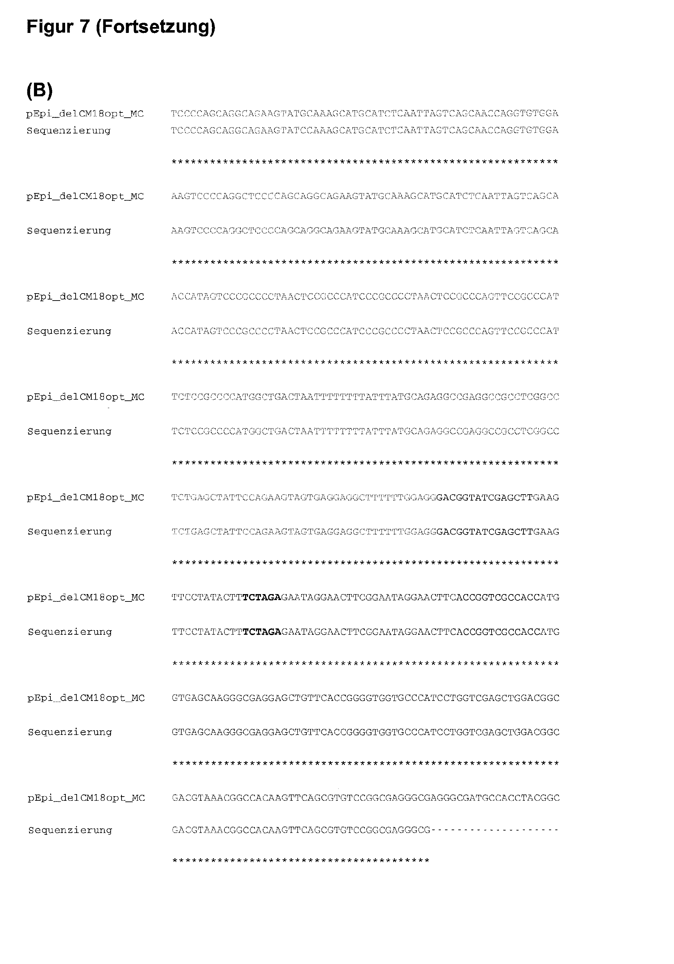

**Figur 7****: Sequenzierung des "*in vitro*"-MiniCircles aus pEpi-delCM18opt**
   (A) Vektorkarte des pEpi-delCM18opt-MC mit sequenzierter Region (roter Pfeil) (B) Vergleich der Sequenz des *in-vitro* MC mit der Sequenz laut Vektorkarte mit dem Programm ClustalW (EMBL-EBI).
   Legende: orange= SV40Promotor/Enhancer; braun= FRT-site; blau= Xbal-Schnittstelle innerhalb der FRT-site; grün= eGFP-Gen; * = Basen-Übereinstimmung.

### Beispiel

### Herstellung und Charakterisierung von MC unter Verwendung eines in vitro Verfahrens mit Ligase und Gyrase

Durch Verwendung von Ligase und Gyrase konnten *in vitro* superhelikale MC erfolgreich hergestellt werden, ohne auf den Einsatz von sequenzspezifischen Rekombinasen oder speziellen Bakterienstämmen zur Replikation der PP und anschließender Induktion der MC Herstellung angewiesen zu sein.

Zu Testzwecken wurde hier ein PP (pEpi-eGFM18 anti HLC) verwendet, das über alle nötigen Elemente verfügt, um mit Hilfe induzierter, sequenzspezifischer Rekombination in MC und MP umgewandelt zu werden. Dadurch war ein direkter Vergleich der beiden Verfahren möglich. Prinzipiell können mit der *in vitro*-Methode bei geeignetem Restriktionsverdau alle Plasmide in MC umgewandelt werden. Eine schematische Darstellung dieses Plasmids ist in Fig. 1 gezeigt.

### 1. Herstellung von MC aus 50 mL Bakterienkultur: Vergleich erfindungsgemäßes (in vitro) und Rekombinase-vermitteltes Verfahren

Die in dieser Anmeldung beschriebenen semi-synthetischen MiniCircle DNA-Vektoren wurden nach dem hier näher beschriebenen Verfahren hergestellt: Das Parentalplasmid - im hier dargestellten Fall pEpi-delCM18opt - bestehend aus MiniPlasmid und MiniCircle-Region, wurde per Elektrotransformation in E.coli XL1 Blue⁽¹⁶⁾ eingebracht. Nach der Selektion von Einzelklonen auf Agarplatten (mit entsprechendem Selektionsmedium) wurde die jeweilige Plasmid-DNA der Klone per Restriktionsverdau und Sequenzierung auf eine korrekte Basenfolge untersucht. Eine langfristige Lagerung der korrekten Klone erfolgte durch Mischen einer 5 ml Übernacht-Kultur mit 87 % Glycerin im Verhältnis 1:1 und Lagerung bei - 20 °C (Glycerin-Stock).

Zur Generierung des Parentalplasmids für die spätere in-vitro-Gewinnung von MiniCircle-DNA wurde etwas Kultur vom Glycerinstock in einen Erlenmeyerkolben mit LB- Selektionsmedium transferiert und bei 37 °C und 180 rpm auf einem Orbitalschüttler ü.N. kultiviert. Zur Gewinnung der Plasmid-DNA (= Parentalplasmid) wurde die Kultur bei 6000 xg für 15 min. abzentrifugiert. Die Präparation der Plasmid-DNA erfolgte mit einem QIAGEN Plasmid-Kit gemäß Anleitung des Herstellers.

Der Minicircle, der von zwei gleichen Restriktionsschnittstellen flankiert wird, wurde per Über-Nacht-Restriktionsverdau aus der Plasmid-DNA geschnitten. Der Restriktionsverdau wurde per Gelelektrophorese (1% Agarosegel) aufgetrennt. Nach dem Gellauf wurde die DNA im Gel durch Methylenblau angefärbt und das Gelstück mit der linearisierten MiniCircle-DNA mit einem Skalpell ausgeschnitten. Die Rückgewinnung des MiniCircles aus dem Gel erfolgte mit dem QIAGEN Gel Extraction Kit gemäß Anleitung des Herstellers.

Die linearisierte MiniCircle-DNA wurde durch eine T4-Ligase bei 16 °C für 16 h religiert. Die Aufreinigung der ligierten DNA aus dem Ligationsansatz erfolgte nach Agarose Gelelektrophorese mit dem QIAGEN Gel Extraction Kit gemäß Anleitung des Herstellers.

Die Überführung des ligierten MiniCircles in den ccc-Zustand wurde durch Inkubation mit einer DNA-Gyrase bei 37 °C erreicht. Die Inkubationszeit richtete sich dabei nach dem gewünschten Grad der Verdrillung und lag zwischen 30 min. und 24 h. Der Ansatz wurde per Gelelektrophorese (1 % Agarose) aufgetrennt. Dabei wurde die DNA mithilfe von Methylenblau angefärbt und danach das Gelstück mit der ccc-MiniCircle-DNA aus dem Gel mit einem Skalpell ausgeschnitten. Die Rückgewinnung des MiniCircles aus dem Gel erfolgte mit dem QIAGEN Gel Extraction Kit gemäß Anleitung des Herstellers.

Die Herstellung der rekombinationsinduzierten MiniCircles aus dem gleichen Parentalplasmid (pEpi-delCM18opt) wie die semi-synthetischen MiniCircles wurde nach dem herkömmlichen Verfahren durch den Einsatz von Rekombinasen wurde wie folgt durchgeführt:
Die FlpE-Rekombinase induzierte Rekombination findet zwischen zwei sog. FRT-Stellen statt, die in diesem Fall die im Parentalplasmid befindliche MiniCircle Sequenz flankieren. Der E.coli-Stamm EL250⁽²⁾ enthält das Gen für die FlpE-Rekombinase im Genom integriert. Dieses Gen steht unter der Kontrolle eines L-Arabinose-induzierbaren Promotors, d.h. dass die Expression dieses Gens erst durch die Zugabe von L-Arabinose in das Kulturmedium angeschaltet wird. Die Induktion findet in M9-Minimalmedium statt, da Glukose oder Saccharose im LB Medium die Aufnahme der L-Arabinose stören würde.

Das Parentalplasmid, bestehend aus MiniPlasmid und MiniCircle-Region, wurde per Elektrotransformation in E.coli EL250 eingebracht. Nach der Selektion von Einzelklonen auf Agarplatten (mit entsprechendem Selektionsmedium) wurde die jeweilige Plasmid-DNA der Klone per Restriktionsverdau und Sequenzierung auf eine korrekte Basenfolge untersucht. Eine langfristige Lagerung der korrekten Klone erfolgte durch Mischen einer 5 ml Übernacht-Kultur mit 87 % Glycerin im Verhältnis 1:1 und Lagerung bei - 20 °C (Glycerin-Stock).

Vor der L-Arabinose-Induktion wurde etwas Kultur vom Glycerinstock in einen Erlenmeyerkolben mit LB-Selektionsmedium transferiert und bei 30 °C für 24 h kultiviert. Anschließend wurde die Kultur bei 3700xg für 15 min. zentrifugiert. Das Pellet wurde in M9-Minimalmedium (1/2 des vorherigen Volumens) aufgenommen bzw. gewaschen und erneut für 15 min. bei 3700xg zentrifugiert. Danach wurde das Pellet in dem ursprünglichen Kulturvolumen in M9-Minimalmedium mit Zusatz von 0,3 % L-Arabinose aufgenommen und in einem Erlenmeyerkolben für 5 h bei 30 °C und 180 rpm auf einem Orbitalschüttler inkubiert. Dadurch wurde die Expression der Flp-Rekombinase induziert, durch die wiederum die Rekombination des Parentalplasmids zu MiniCircle und MiniPlasmid katalysiert wurde (diese Reaktion läuft in den Bakterien nicht quantitativ ab, so dass dabei stets nicht umgesetztes Parentalplasmid in den Bakterien zurückbleibt).

Zur Gewinnung der nicht chromosomalen DNA (= MiniCircle, Parentalplasmid und Miniplasmid) wurde die Kultur bei 6000xg für 15 min. abzentrifugiert. Die Präparation der Plasmid-DNA erfolgte mit einem QIAGEN Plasmid-Kit gemäß Anleitung des Herstellers.

Durch einen Über-Nacht Restriktionsverdau mit einem geeigneten Restriktionsenzym, das ausschließlich in der MiniPlasmid-Region schneidet, wurden das nicht umgesetzte Parentalplasmid und das als nicht benötigtes Beiprodukt entstandene MiniPlasmid linearisiert. Der MiniCircle wurde durch diese Reaktion nicht verändert und blieb deshalb im ccc-Zustand. Der Restriktionsverdau wurde per Gelelektrophorese (1 % Agarosegel+ 0,5 µg/mL Ethidiumbromid) aufgetrennt. Das Gelstück mit der ccc-MiniCircle-DNA wurde auf einem UV-Tisch mit einem Skalpell ausgeschnitten. Die Rückgewinnung des MiniCircles aus dem Gel erfolgte mit dem QIAGEN Gel Extraction Kit gemäß Anleitung des Herstellers.

Das Ausgangsvolumen der Bakterienkultur betrug in beiden Fällen 50 mL.

Wie in Fig. 2 deutlich sichtbar, ist die Ausbeute an MC DNA bezogen auf das Ausgangsvolumen der Bakterienkultur beim erfindungsgemäßen Verfahren etwa 10-15 Mal höher als bei dem Verfahren durch sequenzspezifische Rekombination. Gründe dafür liegen insbesondere in der höheren Ausgangszahl an PP-Kopien pro Bakterium bei dem für das erfindungsgemäße Verfahren verwendeten Bakterienstamm und in der im Vergleich zur Ligation niedrigen Effizienz der sequenzspezifischen Rekombination (deutlich sichtbar an der starken PP Bande im Gel).

### 2. Nachweis des superhelikalen Status von in vitro-hergestelltem MiniCircle

Der superhelikale Status einer DNA gibt an, in welchem Maße die Doppelhelix nochmals in sich selbst verdrillt ist. Dieser "Verdrillungsstatus" gilt als entscheidend für die Effizienz von DNA-Vektoren bzgl. deren Eigenschaft zur Transformation eukaryontischer Zellen mit signifikanter Auswirkung auf Stabilität der Expression und Integration in das Wirtszellgenom.

### 2.1 Superhelikaler Status von aus Bakterien isolierter Plasmid-DNA

Um den Grad der Verdrillung von ccc-DNA zu untersuchen, eignet sich ein Agarosegel, dem Chloroquin in einer bestimmten Konzentration zugesetzt wurde (10). Chloroquin fügt positive "supercoils" in die negative ccc-DNA ein. Außerdem trennt sich die ccc-DNA-Bande je nach Stärke der Verdrillung auf. Zunächst wurde die Methode etabliert. Dazu wurden die ccc-Plasmide pMAXGFP (LONZA, Nucleofector-Kit), CMV-GFP-Parentalplasmid (Plasmid Factory) und pEpi-delCM18opt (Rentschler Biotechnologie) auf einem 0,8 % Agarosegel mit 2,5 µg mL⁻¹ Chloroquin für 15 h bei 2,5 V cm⁻¹ aufgetrennt (Fig. 3).

Sowohl die kommerziellen Plasmid-Proben als auch die selbst-präparierte Plasmid-DNA aus *E*. *coli* XL1 Blue (pEpi-delCM18opt) stellen ein Gemisch aus einem Plasmid mit verschiedenen "Verdrillungsgraden" dar. Aus der Grafik wird deutlich, dass keine einheitliche Verdrillungszahl in Prokaryoten vorherrscht bzw. jedes Plasmid ein eigenes Bandenmuster besitzt. Bei einer Konzentration von 2,5 µg mL⁻¹ Chloroquin läuft ccc-DNA mit einer hohen Verdrillungszahl schneller als mit einer niedrigen Verdrillungszahl. Bei höheren Chloroquin-Konzentrationen verhält sich die DNA umgekehrt.

### 2.2 Vergleich des superhelikalen Status von MC aus ortsspezifischer Rekombination und von erfindungsgemäßen (in vitro) MC

Um die Verdrillung der MiniCircle per ortsspezifischer Rekombination mit dem *in vitro*-MiniCircle vergleichen zu können, wurde die niedrigste einzusetzende DNA-Menge bestimmt, da die Ausbeute an MiniCircle per ortsspezifischer Rekombination sehr gering ist. Dazu wurden die Verdünnungen von 50 - 500 ng des ccc-Parentalplasmids pEpi-delCM18opt verwendet (Fig. 4).

Für die Auftrennung der ccc-DNA reichen 50 ng DNA aus, um das entstehende Bandenmuster mit ImageJ auswerten zu können. Im Folgenden werden der per ortsspezifischer Rekombination in E.coli erzeugte und der *in vitro* MC in Bezug auf ihr Bandenmuster verglichen, um die Eignung der Gyrase bei der *in vitro*-Generierung von ccc-DNA zur Erzielung der verschiedenen Verdrillungszahlen zu zeigen.

Um den Grad der Verdrillung von ccc-DNA zu untersuchen, wurde die Auftrennung der ccc-DNA mithilfe eines Chloroquin-Agarosegels durchgeführt.

Zur Generierung der *in vitro*-MC wurde untersucht, ob die Ligase zusammen mit der Gyrase in einem Reaktionsansatz eingesetzt werden kann. Wie in Fig. 5 (A, Spur 2) erkennbar, ist dies unter den getesteten Reaktionsbedingungen nicht möglich. Die Gyrasebehandlung hatte in diesem Fall keine Wirkung. Die Aufreinigung der Ligation mit anschließender Gyrasebehandlung zeigte dagegen ein positives Ergebnis, d.h. ccc-DNA (Fig. 5, A, Spur 3). Beim Vergleich der beiden MCs ist erkennbar, dass beide MCs ein vergleichbares Bandenmuster aufweisen, beim *in vitro* MC jedoch eine Bande besonders ausgeprägt ist. Ein weiterer Vorteil der neuen Methode ist, dass die Qualität der Verdrillung der *in vitro* MC durch die verwendete Menge an Gyrase und die Inkubationszeit direkt eingestellt werden kann. Im vorliegenden Fall wurde eine große Menge an Gyrase (5 U) eingesetzt und eine lange Inkubationszeit gewählt, um ein gleichmäßiges Bandenmuster zu erhalten, das eine gleichmäßige Verteilung verschiedener Verdrillungsstufen widerspiegelt (Angaben des Gyrase Herstellers New England Biolabs: 1 U Gyrase verdrillt 0,5 µg DNA in 30 min.).

### 3. Überprüfung der Reinheit der in vitro MC Präparationen mittels PCR

Um die höhere Reinheit unseres neuen Verfahrens gegenüber den verschiedenen anderen Verfahren zu zeigen, wurden verschieden hergestellte MC-Präparationen verglichen. Hierzu wurde jeweils eine PCR auf die Miniplasmid-Region und über eine der beiden im PP enthaltenen FRT-Erkennungsstellen - zum Nachweis evtl. vorhandenen Parentalplasmids - durchgeführt (Fig.6). Dazu wurden MC verwendet, die aus dem PP pEpi-delCM18opt generiert worden waren.

Die PCR Reaktionen zum Nachweis von MiniPlasmid und/oder Parentalplasmid Verunreinigungen in der MiniCircle Präparation wurden im in dieser Anmeldung dargestellten Fall wie im Folgenden beschrieben durchgeführt:
PCR1 - Nachweis von MiniPlasmid oder Parentalplasmid: Pro Reaktion wurden 10 pmol an "Primer 3" (TTTTCTGCGCGTAATCTGCT) und "Primer 4" (GTAAAAAGGCCGCGTTGCT) eingesetzt. Diese wurden mit 10 ng MiniCircle Präparation bzw. Parentalplasmid-Kontroll-DNA unter Verwendung der RedTaq-Polymerase (Invitrogen) mittels folgendem Programm zur Amplifikation evtl. Verunreinigungen eingesetzt: 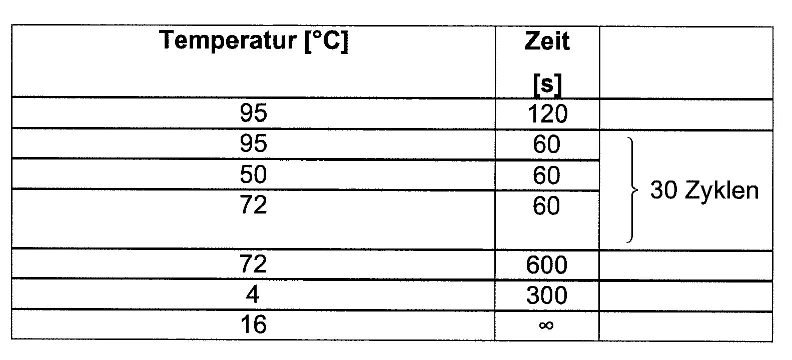 Als Positivkontrolle wurde das zu den MiniCircles korrespondierende Parentalplasmid pEpi-delCM18opt verwendet. Das bei Verunreinigung der Präparation zu erwartende Amplifikat hat eine Größe von 602 bp.
PCR2 - Nachweis von Parentalplasmid: Pro Reaktion wurden 10 pmol an "Primer 1" (GCATGCCATCATGACTTCAG) und "Primer 2" (CGAAACGATCCTCATCCTGT) eingesetzt. Diese wurden mit 10 ng MiniCircle Präparation bzw. Parentalplasmid-Kontroll-DNA unter Verwendung der RedTaq-Polymerase (Invitrogen) mittels folgendem Programm zur Amplifikation evtl. Verunreinigungen eingesetzt:

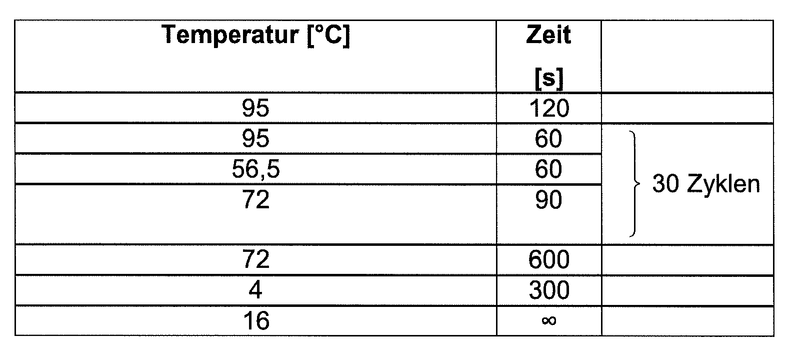

Als Positivkontrolle wurde das zu den MiniCirclen korrespondierende Parentalplasmid pEpi-delCM18opt verwendet. Das bei Verunreinigung der Präparation zu erwartende Amplifikat hat eine Größe von 876 bp.

Überraschenderweise enthält nur der mit der klassischen Methode in *E. coli* EL250 hergestellte MiniCircle Verunreinigungen mit Parentalplasmid und evtl. auch Miniplasmid. Der mit dem neuen Verfahren hergestellte *in vitro-*Minicircle weist keine DNA-Verunreinigungen mehr mit MP oder PP auf.

### 4. Kontrolle auf den korrekten Zusammenbau des in vitro MC durch die Ligase

Durch eine partielle Sequenzierung des *in vitro* MC wurde untersucht, ob dieser auch im Hinblick auf seine Sequenz dem per ortsspezifischer Rekombination generierten MC entspricht. Dazu wurde die Region der nach Rekombination entstandenen FRT-Stelle sequenziert (Fig.7), wobei zunächst die Trennung von MC und MP durch das Restriktionsenzym Xbal erfolgte, sowie anschließend eine Religation durch Zirkularisierung des MC-Fragmentes zum gewünschten *in vitro* MC.

Die Daten der partiellen *in vitro* MC-Sequenzierung ergaben, dass die FRT-Region des *in vitro* MCs der der FRT-Region der Vektorkarte entspricht. Durch die Restriktion mit Xbal mit anschließender Ligation ist, wie bei der ortsspezifischen Rekombination, eine neue FRT-site entstanden. Damit ist eindrucksvoll die Überlegenheit der neuen Methode gezeigt. Die Re-Ligation der linearen MC-Fragmente zu zirkulären *in vitro* MC funktioniert erwartungsgemäß sicher.

### 5. Zusammenfassung der vorliegenden Ergebnisse

Die Daten der vorliegenden Anmeldung zeigen, dass es möglich ist, MC DNA-Vektoren *in vitro* ohne den Einsatz von ortsspezifischen Rekombinasen herzustellen, die in ihren strukturellen Eigenschaften den herkömmlichen durch ortsspezifische Rekombination hergestellten MC entsprechen.

Dabei zeichnen sich die durch die *in vitro* Methode hergestellten MC durch eine signifikant größere Reinheit und das Herstellungsverfahren durch eine deutlich höhere Ausbeute bezogen auf das Ausgangsvolumen der Bakterienkultur aus.

Durch den Verzicht auf die ortsspezifische Rekombination zur Herstellung des MC können prinzipiell alle Plasmid-DNA-Vektoren als Ausgangsmaterial zur MC Herstellung verwendet werden. Eine Klonierung der gewünschten Vektorsequenzen ("Gene of Interest") in spezielle Parentalplasmide mit entsprechenden Rekombinationssequenzen, entfällt bei der *in vitro* Methode.

### Literatur

(1) Bigger Brian W. et al. 2006, Methods of making minicircles, United States Patent Application 20060211117
(2) Broll Sandra 2009, Selbstreplizierende nicht-virale Episomen (Minicircles): Expressionseigenschaften und Etablierung im Zellkern, Dissertation
(3) Broll Sandra et al. 2010, Minicircle Performance Depending on S/MAR-Nuclear Matrix Interactions, Journal of Molecular Biology, Volume 395, Issue 5, 950-965
(4) Che-Kun James C. et al. 1986, DNA supercoiling of recombinant plasmids in mammalian cells, Proc. Natl. Acad. Sci. USA, Vol 83, pp 1641-1645
(5) Chen Zhi-ying et al. 2003, Minicircle DNA Vectors Devoid of Bacterial DNA Result in Persistent and High Level Transgene expression in vivo; Molecular Therapy, Vol. 8, 495-497
(6) Chen Zhi-ying et al. 2004, Silencing of episomal transgene expression by plasmid bacterial DNA elements in vivo, Gene Therapy, Vol. 11, 856-864
(7) Chen Zhi-ying et al. 2005, Improved Production and Purification of Minicircle DNA Vector Free of Plasmid Bacterial Sequences and Capable of Persistent Transgene Expression In Vivo; Human Gene Therapy, Vol. 16, 126-131
(8) Chen Zhi-ying et al. 2010, Minicircle DNA vector preparations and methods of making and using the same, United States Patent Application 20100075401
(9) Esposito Franca et al. 1987, Supercoiling in prokaryotic and eukaryotic DNA: changes in response to topological perturbation of plasmids in E. coli and SV40 in vitro, in nuclei and in CV-1 cells, Nucleic Acids Research, Vol. 15 Num. 13, 5105-5124, 1987
(10) Goldstein et al. 1984, Regulation of bacterial DNA supercoiling: plasmid linking numbers vary with growth temperature, Proc. Natl. Acad. Sci. USA, Vol. 81, 4046-4050
(11) Mayrhofer Peter et al. 2007, Minicircle vector production, United States Patent Application 20070031378
(12) Mayrhofer Peter et al. 2008, Minicircle-DNA production by site specific recombination and protein-DNA interaction chromatography; The Journal Of Gene Medicine, Vol. 10, 1253-1269
(13) Nehlsen Kristina 2004, Molekulare Grundlagen der episomalen Replikation: Charakterisierung zirkulärer, nichtviraler Vektoren; Dissertation
(14) Nehlsen Kristina et al. 2006, Replicating minicircles: Generation of nonviral episomes for the efficient modification of dividing cells, Gene Ther Mol Biol Vol 10, 233-244
(15) Zechiedrich L. et al. 2009, Generation of minicircle DNA with physiological supercoiling, United States Patent 7622252
(16) Bullock WO, Fernandez JM, JM Short. 1987. XL1-blue. A high efficiency plasmid transforming recA Escherichia coli strain with betagalactosidase selection. Biotechniques 5: 376-378

## Patentansprüche

1. Verfahren zur Herstellung eines zirkulären DNA-Vektors in superhelikaler Form, umfassend die Schritte:
(a) Spalten eines Parentalplasmids mit einem oder mehreren Restriktionsenzymen, wobei das Parentalplasmid die Sequenz des DNA-Vektors sowie heterologe Sequenzen enthält, um ein lineares DNA-Vektor-Fragment mit der Sequenz des DNA-Vektors zu erhalten,
(b) Abtrennen des linearen DNA-Vektor-Fragments von anderen Produkten der Reaktionsspaltung,
(c) Ligieren des linearen DNA-Vektor-Fragments, um einen zirkulären DNA-Vektor in relaxierter Form zu erhalten,
(d) Abtrennen des zirkulären DNA-Vektors von anderen Produkten der Ligation,
(e) Verdrillen des zirkulären DNA-Vektors aus Schritt (d) mit einer Gyrase, um einen zirkulären DNA-Vektor in superhelikaler Form zu erhalten, und
(f) gegebenenfalls Aufreinigen des zirkulären DNA-Vektors in superhelikaler Form zur Abtrennung von Nebenprodukten.

2. Verfahren nach Anspruch 1, **dadurch gekennzeichnet, dass** das Parentalplasmid durch Kultivierung einer Wirtszelle, insbesondere einer prokaryontischen Wirtszelle, und Isolierung des Plasmids aus der Wirtszelle gewonnen wird.

3. Verfahren nach Anspruch 2, **dadurch gekennzeichnet, dass** das Parentalplasmid in zirkulärer Form aus der Wirtszelle gewonnen wird.

4. Verfahren nach einem der Ansprüche 1-3, **dadurch gekennzeichnet, dass** ein Parentalplasmid verwendet wird, das frei von Rekombinase-Erkennungssequenzen ist.

5. Verfahren nach einem der Ansprüche 1-4, **dadurch gekennzeichnet, dass** die heterologen Sequenzen des Parentalplasmids die zur Propagation in einer Wirtszelle, insbesondere einer prokaryontischen Wirtszelle, erforderlichen Sequenzen umfassen.

6. Verfahren nach einem der Ansprüche 1-5, **dadurch gekennzeichnet, dass** ein DNA-Vektor hergestellt wird, der im Wesentlichen frei von prokaryontischen Sequenzen ist.

7. Verfahren nach einem der Ansprüche 1-6, **dadurch gekennzeichnet, dass** der DNA-Vektor ein Transgen in operativer Verknüpfung mit regulatorischen Sequenzen enthält.

8. Verfahren nach einem der Ansprüche 1-7, **dadurch gekennzeichnet, dass** ein DNA-Vektor hergestellt wird, der S/MAR-Sequenzen enthält.

9. Verfahren nach einem der Ansprüche 1-8, **dadurch gekennzeichnet, dass** die Restriktionsspaltung in Schritt (a) *in vitro* durchgeführt wird.

10. Verfahren nach einem der Ansprüche 1-9, **dadurch gekennzeichnet, dass** Schritt (b) eine Gelelektrophorese, insbesondere eine Agarose-Gelelektrophorese, umfasst.

11. Verfahren nach einem der Ansprüche 1-10, **dadurch gekennzeichnet, dass** die Ligation in Schritt (c) *in vitro* durchgeführt wird.

12. Verfahren nach einem der Ansprüche 1-10, **dadurch gekennzeichnet, dass** das Verdrillen in Schritt (e) *in vitro* durchgeführt wird.

13. Reagenzienkit zur Herstellung eines zirkulären DNA-Vektors in superhelikaler Form, insbesondere zur Verwendung in einem Verfahren nach einem der Ansprüche 1-12, umfassend
(a) eine Ligase,
(b) eine Gyrase, und
(c) ggf. ein oder mehrere Restriktionsenzyme.

14. Präparation eines MiniCircle DNA-Vektors in superhelikaler Form, **gekennzeichnet durch** das Fehlen von Nebenprodukten nach PCR, insbesondere linearem oder zirkulärem Miniplasmid und/oder Parentalplasmid.

15. Präparation nach Anspruch 14, erhältlich durch das Verfahren nach einem der Ansprüche 1-12.

16. Verfahren zur Herstellung von superhelikalen zirkulären DNA-Vektoren, umfassend eine Restriktionsspaltung *in vitro,* eine Ligation *in vitro* und eine Verdrillung mit Gyrase *in vitro.*

17. Verfahren zur Herstellung von superhelikalen MiniCircle DNA-Vektoren ohne den Einsatz orts-(sequenz-) spezifischer Rekombinasen wie etwa FLP.
